# EUROPEAN PATENT APPLICATION

(11) **EP 2 803 327 A1**
(43) Date of publication of application: **19.11.2014**
(21) Application number: 14180580.4
(22) Date of filing: 14.07.2008
(51) Int. Cl.: A61B 17/70, A61B 17/00

(54) **Systems for spinal stabilization**

(30) Priority: 13.07.2007 US 959456 P
(62) Divisional of application: 08794499.7
(71) Applicant: Frey, George, Englewood, CO 80113 (US)
(72) Inventor: Frey, George, Englewood, CO 80113 (US)
(74) Representative: Heyer, Volker

(57) **Abstract**

Spinal stabilization systems are disclosed having spanning portions extending between and securable to pedicle screw assemblies, the spanning portions have stiffness characteristics that may be variable or selectively adjustable, and/or have non-linear behavior with respect to force versus distortion. Additionally, the systems may utilize a plurality of spanning portions in which two or more of the spanning portions have different stiffness characteristics.

## Description

### CROSS-REFERENCE TO RELATED APPLICATION(S)

The present application claims the benefit of U.S. Provisional Patent Application No. 60/959,456, titled "Systems and Methods for Spinal Stabilization," and filed July 13, 2008, the entirety of which is incorporated by reference herein.

### FIELD OF THE INVENTION

The invention relates to methods and systems for spinal stabilization and, in particular, to methods and systems allowing for variability of the mechanical behavior of spinal stabilization system and, more particularly, to such methods and systems that allow a user-surgeon to select or adjust the mechanical behavior of a spinal stabilization during implantation, as well as extra-corporeally after implantation.

### BACKGROUND

Spinal stabilization systems take a variety of forms. Typically, these systems would more generally be described as spinal immobilization systems as the intent is for relative movement between adjacent vertebral sections to be prevented. For instance, most intervertebral implants are known as fusion devices as they are designed to form a permanent or semi-permanent bond with the adjacent vertebrae so that the vertebrae themselves are referred to as "fused."

Other spinal stabilization systems involve the use of anchors secured with a plurality of vertebrae and spanning members between the anchors. Such devices are often referred to by the portion of the vertebra to which the anchors secure. For instance, a laminar stabilization system utilizes anchors, typically hooks, secured with the lamina of a vertebra. As another example, a stabilization utilizing anchors in the form of a screw is often referred to as a pedicle screw system, as the screws themselves are driven into the pedicle portion of the vertebra.

Generally speaking, the spanning member is the least considered part of this type of system. A surgeon's choices for spanning members are virtually limited to selecting either a rod or a bar, the length of the spanning member, and a cross-sectional dimension such as the rod's diameter.

It should be noted that there are particularized types of rod a surgeon can select. Generally, however, these rods are limited in use to an entire system, and the deviation from the standard rod provided by these rods is not for mechanical behavior characteristics, instead being for cooperation with the other particularized features of a specific stabilization system.

Other than portions of the above discussion, the term "stabilization system" is meant to refer only to spinal stabilization systems that attach to one or more vertebrae in a manner that does not affect or interfere with the intervertebral space, nucleus, or annulus. Accordingly, laminar or pedicle systems or the like are each intended to be encompassed by the term "stabilization system."

In general terms, a stabilization system is implanted through an open and retracted incision by securing at least one anchor on an inferior vertebra and at least one anchor on a superior vertebra. It should be noted that the medical community is continuing to develop minimally invasive surgical techniques for implantation of such devices. Typically, a pair of anchors is secured with each of the vertebrae, and typically the vertebrae are adjacent. In some forms, the stabilization system may span three or more vertebrae and be secured with any two or more of the vertebrae.

Spanning members are then secured with the anchors. This commonly requires forcing rods into a yoke secured with each of the anchors. In some forms, the anchor and yoke are of a type referred to as "polyaxial" by their ability to pivot relative to each other so that a channel in the yoke for receiving the rod becomes aligned in an optimal orientation for receiving the rod. The spanning members are usually then secured in and with the yoke with a securement in the form of a cap that is received in an upper portion of the yoke channel.

The entire stabilization system is generally highly rigid. Once the rod is secured therein, the cap and the yoke frequently distort or deface the surface of the rod via the pressure exert to secure the rod therein. This prevents movement of the rod within (such as rotation) or relative to the yoke and anchor (such as longitudinal sliding). The rod itself is formed of a high modulus of elasticity metal, and its mechanical behavior displays little elasticity.

Stabilization systems have been developed to allow some motion in one or more directions. Generally, motion of a normal, healthy spine includes anterior-posterior flexure, lateral flexure, and rotation, or any combination of these. Due to disease, damage, or natural defect, the purpose of the stabilization system may vary. Depending on such purpose for the stabilization procedure utilizing the stabilization systems, motion in one or more directions may be preferred to a rigid system.

It is also known that there are medical detriments that can arise from full immobilization. For instance, it is know that a lack of pressure (i.e., stress, or weight) on bones can result in a decrease in density. An expression known as Wolfs law describes the benefits of pressure on bones or bone fragments as they are healing, benefits that can be negated by an overly rigid spinal stabilization system. It is also suspected that intervertebral structures may suffer from a lack of use resulting from rigid systems. Additionally, full immobilization can result in overstressing of adjacent areas, thus producing adjacent segment degeneration.

Accordingly, some stabilizations systems have been designed to allow the portion of the spine to which the system is secured to bend itself. For instance, the ends of a spanning member may be curved relative to each other due to motion in some directions, like a cylindrical rod being curved.

A complicated example of stabilization system permitting some bending motion is described in U.S. Patent No. 5,961,516, to Graf. In simple terms, the system of the '516 patent includes anchors for respective vertebrae and a spanning structure connected with the anchors. The spanning structure includes a ball joint between two portions, and a "compressible" body acting as a shock absorber. The various components of the system of the '516 patent must clamp tightly and utilize friction in order to resist free movement. Over time, such friction results in wear to the components, which in turn may lead to reduced performance of the components, and revision surgery, or fragments of the components being free in the patient's body. It is also known that implantation of an elastomeric/polymeric compressible member is difficult as the material is prone to release of polymeric byproducts and is prone to chemical and mechanical degradation.

Another direction of motion that ideally is accommodated is that which shifts the anchors themselves relatively and directly in line with the spanning structure. The '516 patent purports to provide a system that allows spinal motion in all directions, only the compressible member allows the spanning structure itself to shorten; additionally, the compressible member is not shown as being able to expand for the spanning structure being lengthened.

Once implanted, the stabilizations system are generally constant in their behavior characteristics, other than changes due to wearing of components or the like. To be specific, a surgeon may select a specific diameter for a rod to span between two anchors, and the diameter and material can be selected for their mechanical properties. The surgeon may also determine either a length of the rod or a distance between the anchors based on how the rod is secured with the anchors. However, the selection of the rod diameter is quantized as it is a specific size, and the surgeon is unable to adjust the exact diameter during a procedure other than to select from specific, predetermined diameters. Subsequent to the surgical procedure, the surgeon is unable to adjust the distance between the anchors without a further, revision surgical procedure, which would also be required if a surgeon were to determine a different diametrally-sized rod would be preferred (such as to increase or decrease the flexure of the spanning structure).

In the selection of the stabilization systems discussed, a surgeon is not provided with sufficient implant options for selecting a desired amount of permitted motion. For instance, a surgeon's choice in implanting a pedicle screw system is generally limited to the cross-sectional size of the rod spanning between the pedicle screw assemblies, and larger rods require a larger yoke provided on the pedicle screw for receiving the rod therein. Even using systems that are designed to permit some degree of motion, such systems do not provide a surgeon the ability to optimize the motion permitted based on a particular patient, they do not allow a surgeon to adjust the mechanical behavior of the system through a linear range, and they do not allow a surgeon to adjust the mechanical behavior without full-scale revision surgery.

Accordingly, there has been a need for improved spinal stabilization systems.

### SUMMARY

In accordance with an aspect, an orthopedic device is disclosed to provide stabilization of the spinal column between anchorage locations on a minimum of two vertebral bodies comprising structural member(s) or spanning portions between each anchorage point, the device or system having the ability to provide stiffness, and the stiffness being variable in longitudinal and transverse planes relative to the spinal column or vertebral bodies.

The stiffness of the structural member(s) can be varied by adjustment of cross-sectional area properties. The stiffness of the structural member(s) can be varied by adjustment of helical coil spring tension / compression. The stiffness of the structural member(s) can be varied by adjustment of hydraulic pressure or volume. The stiffness of the structural member(s) can be varied by adjustment of pneumatic pressure. The stiffness of the structural member(s) can be varied by combining materials of differing properties.

An orthopedic device of the present invention may comprise at least two structural members, one of which has an outer cross-sectional profile that is smaller than the inner cross-sectional area of the other and is able to seat inside another structural member, the members being retained with a first end secured with a first vertebral body, and a second end operatively fixed with a second vertebral body. The orthopedic device may comprise at least two structural members, each of which has a non-uniform longitudinal cross-sectional area.

Structural members may have the ability to be retained at anchorage positions in any orientation along the transverse plane and, furthermore, have the ability to interface with one another in orientation along the transverse plane.

The orthopedic device may comprise at least two structural members whose geometry allows the two to be mated together and received into each anchorage point for securement at each level.

The orthopedic device may comprise a length appropriate helical coil spring with corresponding attachment fittings at each end. Each attachment fitting may have the ability to be secured to each attachment point. While securely attached to the helical coil spring, each fitting has the ability to translate radially (or rotationally) with respect to the anchorage point which effectively changes the geometric condition of the helical coil spring (reduce or enlarge the diameter). A length appropriate cylindrical rod may be located concentrically with the helical coil spring.

An orthopedic device which may comprise at least one helical coil spring (compression) concentrically located inside an additional helical coil spring (extension) the outer helical coil spring anchored to each vertebral body the inner helical coil spring retained to each anchorage point at each vertebral body. The anchorage points may interface with each helical coil spring having longitudinal adjustability, and additionally have the ability to receive a cylindrical rod concentrically to both helical coil springs for another opportunity to alter the stiffness of the device

An orthopedic device may comprise a pressure vessel which is placed in the vicinity of and attached to each anchorage point, the pressure vessel having two or more independent, directional flow restricting valves. One valve may be for allowing fluid delivery into the pressure vessel, while another valve may serve to permit fluid exiting the pressure vessel. The valves may be disposed in a plurality of configurations including being integral with the structural members, being disposed on an external line thereto, or being disposed with a reservoir and system for adjusting the pressure/volume of the pressure vessel, any of such components (i.e, the valve, line, reservoir, and pressure system and actuator therefor) being disposed either subcutaneous or extracorporeal.

An orthopedic device may comprise a piston/cylinder configuration which is oriented longitudinally and secured to each anchorage point on each vertebral body, the piston having flow orifices of which the same could be adjusted to vary the volumetric flow rate and, ultimately, device stiffness.

A orthopedic device may comprise a pressure vessel which is located longitudinally between and attaches to each anchorage point, the pressure vessel additionally having an integrated reservoir which could be accessed post operatively for the purpose of introducing or removing working fluid to/from the pressure vessel.

An orthopedic device may comprise a pressure vessel which is located longitudinally between and attaches to each anchorage point, the pressure vessel having two independent, directional flow restricting valves. The first valve would allow a pressurized gas to be delivered inside the pressure vessel. The second valve would allow pressurized gas to exit the pressure vessel.

In an aspect of the invention, a spinal stabilization system securable with a plurality of vertebrae is disclosed including at least one anchor for at each of least two vertebrae, and a spanning structure extending between and securable with the anchors, wherein the spanning structure has an adjustable mechanical performance characteristic.

In some forms, the mechanical performance characteristic is a bending stiffness. The bending stiffness may be adjustable in orientation relative to the vertebrae. The bending stiffness may be adjustable in anterior, posterior, lateral, and torsional modes. The bending stiffness may be selected by selection of cross-sectional areas of the spanning structure. The bending stiffness may be selected by selection of differing materials for the spanning structure.

In some forms, the spanning structure may include an outer member and an inner portion, wherein the bending stiffness may be selected by selection of the inner portion. The inner portion may be provided after securing the outer member with the anchors. The inner portion may be comprised of a plurality of inner components, and the bending stiffness may be selected by selecting a number of the components to be disposed within the outer member. The the bending stiffness may be adjusted by removal or addition of the inner components. The the bending stiffness may be adjusted by orientation of the inner portion relative to the outer member. At least one of the outer member and the inner portion may have eccentrically positioned regions of reduced cross-sectional area, and rotation of the regions provides a direction for lowered stiffness.

In some forms, the mechanical performance characteristic is a compression/expansion stiffness. The spanning structure may include a spring including a plurality of coils. The stiffness may be adjustable by adjusting at least one physical characteristic of the spring. The physical characteristic may include at least one of the number of coils, the diameter of the coils, and the length of the spring. The coil spring may be an outer member, and the spanning structure may further include an inner portion, wherein the coil spring may provide a selectable and adjustable compression/expansion stiffness, and the inner portion may provide a bending stiffness.

In some forms, the spanning structure may includes a pair of springs each having a plurality of coils, wherein a first of the springs may provide a compression characteristic and a second of the springs may provide an expansion characteristic. The spanning structure may further include an inner portion, wherein one of the springs of the pair forms an outer spring, the other of the springs forms an inner spring, and the inner portion is disposed within the inner spring, the inner portion providing a bending stiffness.

In some forms, the spanning structure may include a piston assembly compressible and expandable along a longitudinal axis thereof. The piston assembly may be provided with compressible gas. The piston assembly may be provided with substantially incompressible fluid. The piston assembly may be provided with a damper.

In some forms, the piston assembly is provided with fluid of mixed phases, a portion of the fluid being compressible gas and a portion of the fluid being incompressible liquid.

In some forms, the piston assembly is provided with fluid, and the amount of fluid may be adjusted to adjust the mechanical performance characteristics. The system may further include a reservoir for fluid, wherein the piston assembly communicates with the reservoir, and the mechanical performance characteristics may be adjusted by increasing the fluid in the piston assembly by delivering fluid thereto from the reservoir and may be adjusted by decreasing the fluid in the piston assembly by delivering fluid therefrom to the reservoir. The the piston assembly and reservoir may be connected via at least two one-way valves for fluid transfer therebetween. The reservoir may be a compressible bladder implanted subcutaneously.

In another aspect, a spinal stabilization system securable with a plurality of vertebrae is disclosed including at least one anchor for at each of least two vertebrae, and a plurality of spanning structures extending between and securable with the anchors, each spanning structure having an adjustable mechanical performance characteristic.

In some forms, each of the spanning structures is adjusted to impart a different stiffness characteristic between its respective anchors.

In some forms, the mechanical performance characteristic of the spanning structures may be adjusted after being secured with the anchors.

In some forms, the mechanical performance characteristic for at least one of the spanning structures is a bending stiffness, and the mechanical performance characteristic for at least one of the spanning structures is a compression/expansion stiffness.

In another aspect, a spinal stabilization system securable with a plurality of vertebrae is disclosed including at least one anchor for at each of least two vertebrae, and spanning structures extending between and securable with the anchors, the spanning structure having an adjustable mechanical performance characteristic, wherein the mechanical performance characteristic is adjustable after the spanning structure is secured with its respective anchors.

In some forms, at least one spanning structure mechanical performance characteristic is adjustable via a percutaneous incision in a patient's skin.

In some forms, at least one spanning structure is adjustable via an end thereof.

In some forms, the system may be adjusted via an implanted key or tool without an incision.

In some forms, at least one spanning structure mechanical performance characteristic is adjustable via a hypodermic needle.

In some forms, at least one spanning structure includes a piston assembly, and the system further including a reservoir for fluid, wherein the piston assembly communicates with the reservoir, the mechanical performance characteristics of the piston assembly being adjustable by increasing the fluid in the piston assembly by delivering fluid thereto from the reservoir and adjustable by decreasing the fluid in the piston assembly by delivering fluid therefrom to the reservoir. The reservoir may be a compressible bladder implanted subcutaneously.

### BRIEF DESCRIPTION OF THE DRAWINGS

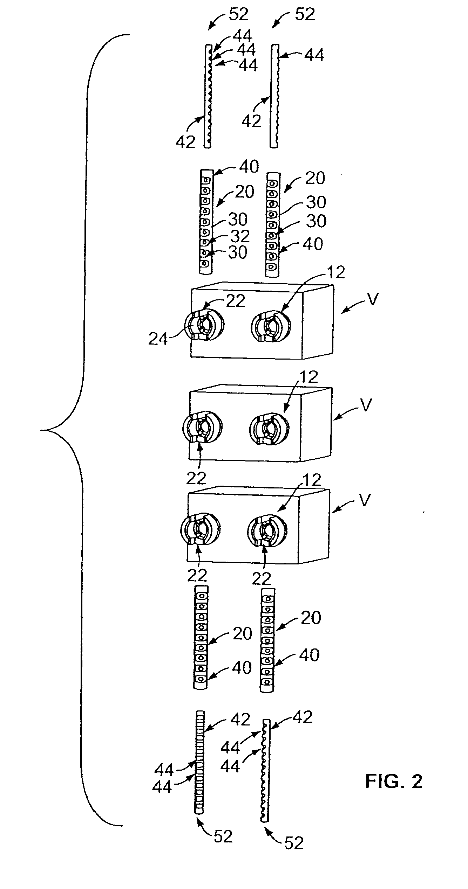

In the Figures, Fig. 2 is a perspective view of a first form of a spinal stabilization system secured with a plurality of representative adjacent vertebrae, the stabilization including a plurality of anchors in the form of pedicle screws and a plurality of spanning structures connecting the anchors, the spanning structures having a selectable and adjustable stiffness in bending or flexure provided by portions of reduced cross-sectional area;
Fig. 2 is an exploded view of the stabilization system and vertebrae of Fig. 1 showing the spanning structures having an outer shell portion and an inner core portion, the shell and core each having portions of reduced cross-sectional area and being positionable relative to each other and to the anchors to provide a desired stiffness in a direction or region for the stabilization system;
Fig. 3 is a top plan view of the stabilization system and vertebrae of Fig. 1 showing the spanning structures received within channels of yokes of the an anchors;
Fig. 4 is a exploded view of the stabilization system and vertebrae corresponding to Fig. 3 showing the reduced cross-sectional area portions of the cores having different orientations relative the shell reduced cross-sectional areas, as well as the anchors of the stabilization system, to provide different stiffness or mechanical performance characteristics to the different spanning structures;
Fig. 5 is a side elevational view of a pair of anchors secured with a vertebra in cross-section, and of spanning structures of the stabilization system of Fig. 1 positioned for securement in the yoke channel thereof, an end of the spanning structure having structure for cooperating with a key or tool for adjusting the position of the core relative to the shell;
Fig. 6 is a representative side elevational view showing an implanted stabilization system having a layer of flesh covering the stabilization system, and access passages through the flesh provided by separate incisions, the access passages allowing access to end of spanning structures of the stabilization system;
Fig. 7 is a representative view of a form of a stabilization system having spanning structures formed of different materials to provide different moduli of elasticity thereto;
Fig. 8 is a perspective view of a form of a stabilization system secured with representative adjacent vertebrae, the stabilization system including spanning members that are provided as multiple pieces joined in the yoke of the anchor to provide different stiffness characteristics between different vertebral levels;
Fig. 9 is a side elevational view of the stabilization system of Fig. 8 showing spanning structures of an upper vertebral level having a greater cross-sectional thickness than spanning structures of a lower vertebral level;
Fig. 10 is a partially exploded view of the stabilization system of Fig. 8 showing a portion of the spanning structure of the upper vertebral level removed, and showing unitary structures disposed in yokes for both the upper and lower vertebral levels;
Fig. 11 is a top plan view of a form of a stabilization system secured with representative adjacent vertebrae, the stabilization system having spanning structures including spring coil portions securable with the channels of the yokes and having end fixtures that are graspable or manipulable with a tool for rotating the end fixtures to alter the stiffness characteristics of the spanning structures;
Fig. 12 is an exploded perspective view of a form of the stabilization system of Fig. 11 showing rod-like central core portions receivable within the coil portions of the spanning structure;
Fig. 13 is a side elevational view of a form of a spanning structure for use with anchors, the spanning structure having a outer sheath or casing which permits addition or removal of core strands therewithin for providing a selected stiffness to the spanning structure;
Fig. 14 is a perspective view of a form of a stabilization system secured with representative adjacent vertebrae, the stabilization system having anchors with posts for engaging with spanning structures having coil springs with end loops;
Fig. 15 is a side elevational view of the stabilization system of Fig. 14;
Fig. 16 is an exploded perspective view of a form of the stabilization system of Fig. 14 showing the coil springs as outer coil springs, showing inner coil springs, and showing central rod-like core members for providing desired stiffness characteristics to the spanning structures;
Fig. 17 is an exploded view of an anchor of Fig. 14 showing a nut for securing the post within a recess of the anchor base, a bore in the post for receiving a core member, and a groove in the post for receiving an end loop of an outer coil spring;
Fig. 18 is a perspective view of a stabilization system secured with representative adjacent vertebrae, the stabilization system including spanning structures having piston assemblies selectively pressurized with fluid such as gas;
Fig. 19 is a top plan view of the stabilization system of Fig. 18;
Fig. 20 is a perspective view of a stabilization system secured with representative adjacent vertebrae, the stabilization system including spanning structures having piston assemblies selectively filled with fluid such as liquid;
Fig. 21 is a top plan view of the stabilization system of Fig. 20; and
Figs. 22A-22C are cross-sectional views of spanning structures for use in stabilization systems having varying spring and stiffness characteristics along their length.

### DETAILED DESCRIPTION

In accordance with aspects of the present invention, a plurality of forms and embodiments of spinal stabilization systems are depicted in the Figs. In a variety of manners, these forms provide a user-surgeon with a range of choices for the motion that is permitted for spanning structures of the spinal stabilization system, the mechanical properties of the spanning structures including flexure, torsion, and/or compression and expansion, with linearly selectable mechanical properties, provide a surgeon with spanning structures that can provide a range of mechanical properties while being used with identical yokes of anchors, allow the surgeon to adjust the mechanical properties in situ, and allow the surgeon to adjust the mechanical properties post-operative without full-scale surgical revision.

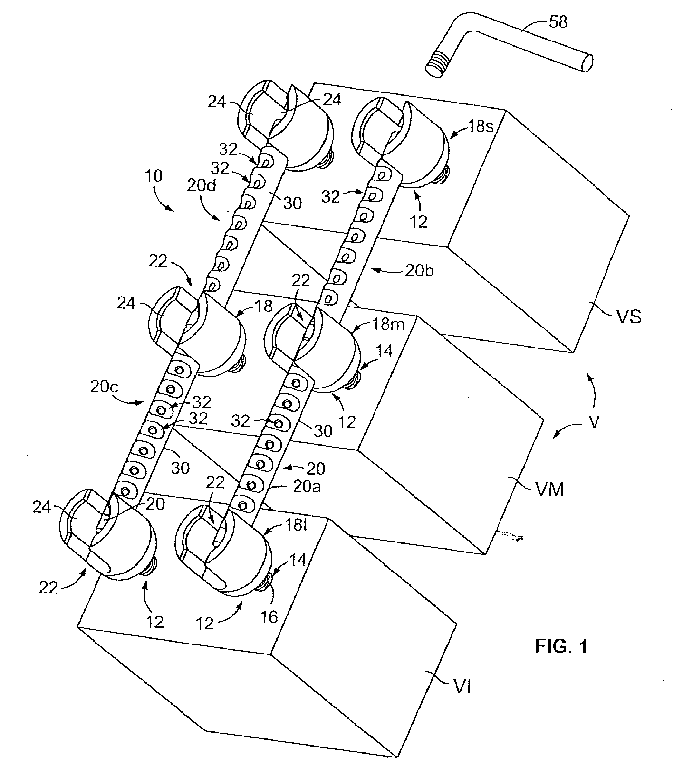

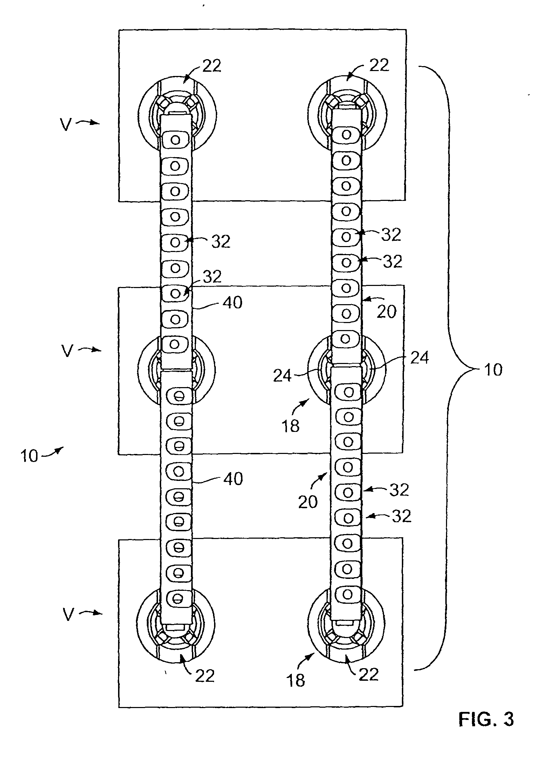

Referring to Figs. 1-5, a first form of a spinal stabilization system 10 of the present invention is illustrated secured with a plurality of representative vertebrae V. As illustrated, the vertebrae V include an inferior vertebra VI, a medial vertebra VM, and a superior vertebra VS. The stabilization system 10 includes a plurality of anchors 12 so that a pair of anchors 12 is provided for each vertebra V, as is well-known in the art. Each anchor 12 includes a screw 14 having a threaded shank 16 received in its respective vertebra V and includes a yoke 18. In some forms, the yoke 18 and shank 16 may be fixed relative to each other, such as by the anchor 12 being a unitary component or by being forming integral. In other forms, the anchor 12 may be a poly-axial anchor so that the yoke 18 may be oriented in a desirable manner once the anchor shank 16 is secured with the vertebra V.

The stabilization system 10 includes spanning structures 20 for connecting the vertebra V to control the relative movement therebetween. Each yoke 18 includes a channel 22 into which one or more spanning structures 20 is received for securement therewith. Once a spanning structure 20 is properly seated in the channel 22, a securement (not shown) generally referred to as a cap is driven atop the spanning structure 20 such as by being threaded into arcuate recesses 24 of the yoke 18 and to the sides of the channel 22.

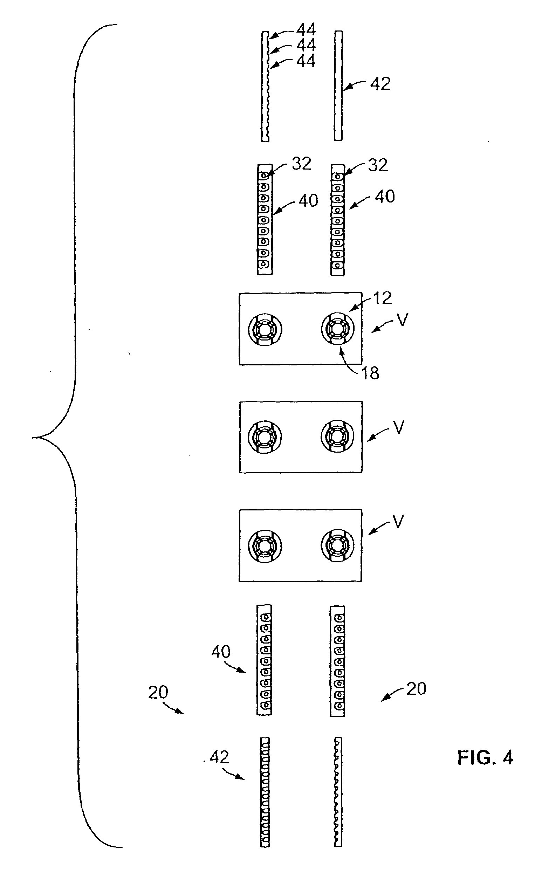

As best seen in Figs. 2 and 4, each spanning structure 20 is generally rod-like with an outer surface 30 with a plurality of cut-outs or scallops 32. The scallops 32 provide stress concentrators or, alternatively viewed, regions of lower stiffness for the spanning structure 20. When the spanning structure 20 is secured with the yokes 18, the scallops 32 are oriented in a direction in which it is desired to permit greater flexure between the anchors 12 to which the spanning structure 20 extends. To be clear, the scallops 32 are areas of reduced cross-sectional area that are eccentrically positioned relative to the central longitudinal axis of the spanning structure 20 so that orientation of the spanning structure 20 provides a distinct direction of lowered stiffness, and so that rotation of the spanning structure 20 alters the direction of lowered stiffness.

As can be seen, a first spanning structure 20a is secured between a first yoke 181 secured with the inferior vertebra VI and with a second yoke 18M secured with the medial vertebra VM while a second spanning structure 20b is secured between the second yoke 18M and a third yoke 18S secured with the superior vertebra VS. When secured, the scallops 32 of the first and second spanning structures 20a, 20b may have different radial orientations such that the flexure mechanical characteristics between the first and second yokes 181 and 18M are different than the flexure mechanical characteristics between the second and third yokes 18M and 18S.

It should also be recognized that the first spanning structure 20a cooperates with a third spanning structure 20c while the second spanning structure 20b cooperates with a fourth spanning structure 20b to define the mechanical properties between their respective vertebrae V; thus, varying the orientations of scallops 32 for each of the four spanning structures 20a-20b serve to provide at least some of the mechanical properties for the stabilization system 10 as a whole. It should also be noted that the materials of the different spanning structures 20a-20b may be varied to provide or influence the mechanical properties of each.

In a further form of the spanning structure 20, the scallops 32 are formed on a shell member 40, and a core member 42 is received within the shell 40. In various forms, the core 42 may be of like or dissimilar materials to influence the mechanical properties to provide varying selected or selectable flexure properties, for instance.

In a preferred form, the core 42 also includes scallops 44 along its length, as best seen in Figs. 2 and 4. When the core 42 is received within the shell 40, the core scallops 44 may be aligned (or misaligned) to varying degrees with the shell scallops 32. As should be evident, when the sets of scallops 44, 32 are aligned, such augments the flexure characteristics and, more appropriately, lessens the stiffness of the spanning structure 20 as a whole in a particular direction. When the scallops 44, 32 are largely misaligned, the decrease in stiffness provided by the different scallops 44, 32 is aligned in first and second directions. For the scallops 44, 32 merely being partially overlapping or relatively juxtaposed, the decrease in stiffness is distributed over the region between and including the scallops 44, 32. It should be noted that the scallops 32, 44 may be aligned or misaligned in both a radial direction (i.e., orientation in a 360 degree sweep) and in an axial direction.

The alignment of the scallops 32, 44 may be selected at any time prior to, during, or after implantation (securement in the yokes 18), as well as after the surgical procedure itself. To promote such adjustment, the core 42 may be provided with structure 50 on one or more ends 52 for engaging and rotating the core 42 relative to the shell 40.

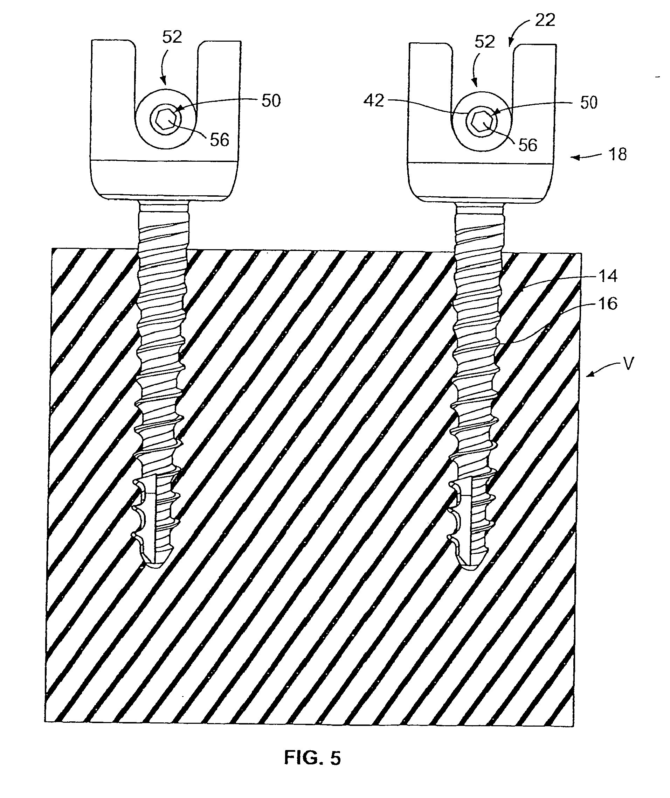

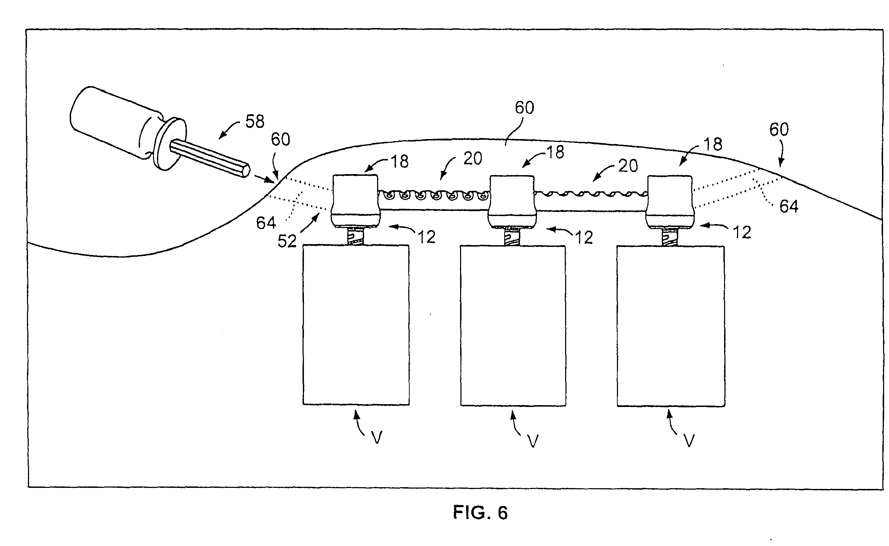

As can be seen in Fig. 5, the core 42 includes a socket 56 shaped for receiving a key 58 (not shown). As an example, the socket 56 may be hexagonal (Fig. 5) for receiving a hexagonal key 58 (Fig. 6). In other forms, the key 58 may have a hook (not shown) or the like for axially advancing or withdrawing the core 42 along the axial direction of the shell 40. In another form, the socket 56 may include a section of internal threading for threadably receiving the key 58, the key 58 having slightly undersized threading (Fig. 1) for easy thread-receipt and effecting rotation in a single direction when fully advanced in the socket 56. Due to the threaded connection, such key 58 enables axial forces to be applied to the core 42 to advance/withdraw the core 42 within the shell 40.

The scallops 32,44 may be cut at an oblique angle relative to a circumference of the shell 40 and/or core 42 so that the scallops 32, 44 may also facilitate or enable torsional distortion thereof. The depth, frequency, and/or size of the scallops 32, 44 may be varied along the length of the shell 40 or core 42 so that the "spring equation" of the spanning structure 20 is non-linear, that is, so that the force required to achieve a certain amount of bending to the spanning structure 20 increases as the bending increases. Instead of the scallops 32, 44, either or both of the shell 40 and/or core 42 may simply be given a non-circular cross-section so that the bending characteristics are not the same throughout a 360 degree sweep.

Turning now to Fig. 6, the spinal stabilization system 10 is depicted as implantated with a layer 60 of a patient's flesh (including the surface skin) located atop the stabilization system 10. As can be seen, a small incision 62 may be made in the layer 60 to provide a passage or access 64 to the end 52 of a spanning structure 20. The key 58 may be inserted through the small incision 62 and the access 64 for connection with the spanning structure 20 socket 56. Accordingly, a major revision surgical procedure is not necessary to alter the mechanical performance characteristics (i.e., flexure or stiffness of the spanning structures 20), as such can be done with a minor procedure. It should also be noted that the core 42 may be entirely removed from the shell 40, which would also permit a new core 42 with greater or lesser stiffness to replace the previous core, all without having to remove the securements (i.e., caps) from the yokes 18.

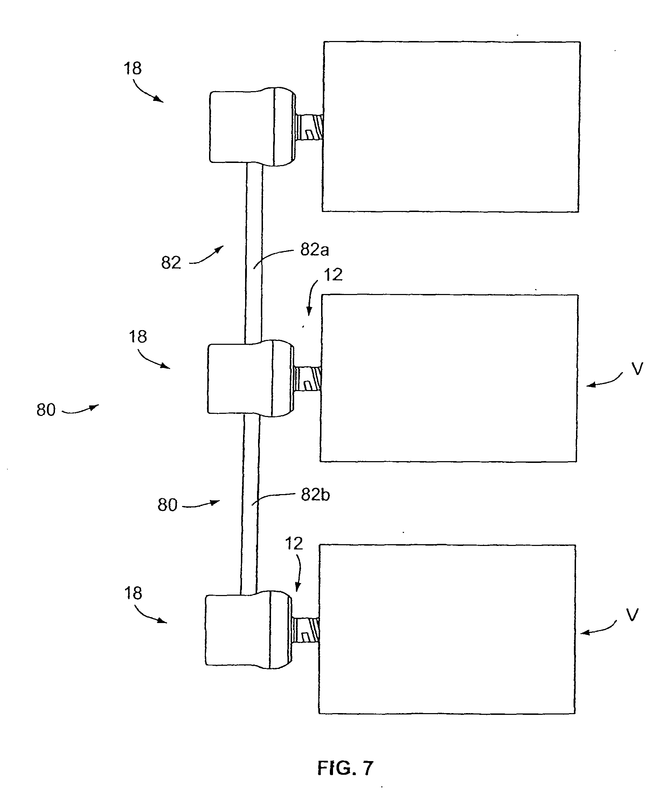

As discussed above, the materials for the spanning structures 20 may be varied to provide different flexure or mechanical performance characteristics. Turning to Fig. 7, a form of a spinal stabilization system 80 is depicted similar to that of Figs. 1-6, though simplified to illustrate spanning structures 82 and, in particular, to depict a first spanning structure 82a having a first modulus of elasticity and a second spanning structure 82b having a second modulus of elasticity that is different from the first, the modulus of elasticity determined by the material from which each spanning structure 82a, 82b is formed. As noted above, in the event a pair of spanning structures 82 is used in tandem to span between two vertebrae V, such as adjacent vertebrae V, the flexure characteristics are determined by a combination of the elastic moduli of the two spanning structures 82 of the pair.

It should be noted that reference to flexure characteristics and mechanical performance characteristics, as used herein, are meant to refer to how a spanning structure and/or a stabilization system performs under load, based on inherent materials properties and structural geometry. While in biomechanics, flexure and extension are generally thought of as being opposite, with respect to curving or bending of a spanning structure, these terms are one and the same. Additionally, these terms are intended in a broad manner to also include torsional distortion or twisting. Modulus of elasticity or elastic modulus is an inherent property of the material, regardless of shape or geometry. While stiffness and modulus of elasticity are typically thought of as linear descriptions of mechanical behavior dependent on shape and material, thereby equating them to a spring equation having a spring constant K (i.e., Force = K x Change in Length), it should be noted that these terms herein encompass a non-linear description of mechanical behavior such that force and distortion are not in direct proportion.

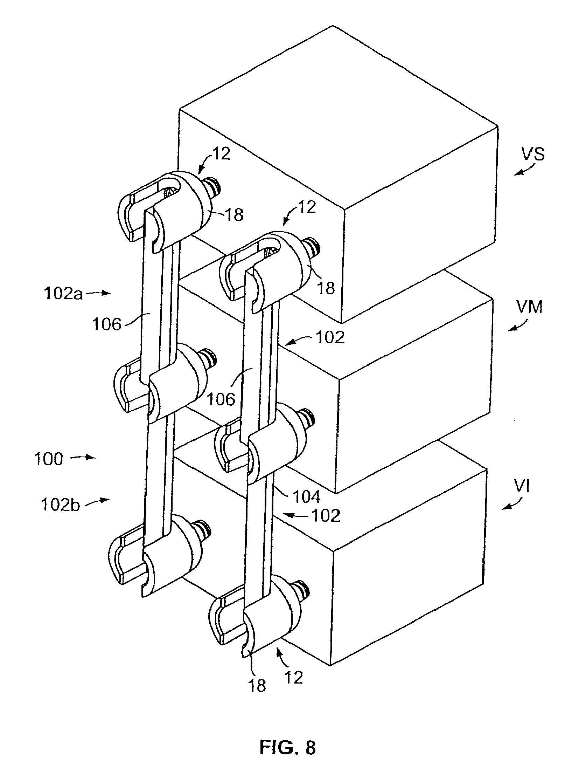

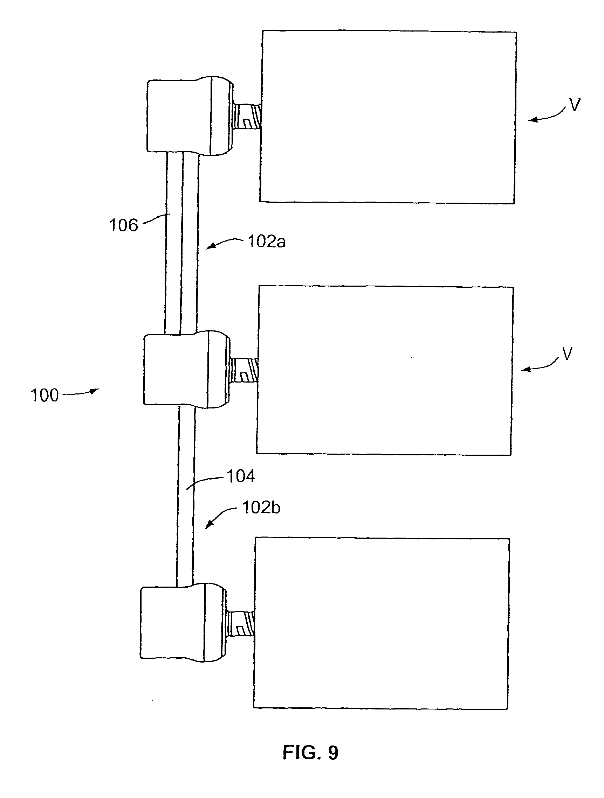

Turning now to Figs. 8-10, a further form of a spinal stabilization system 100 is illustrated having spanning structures 102 with different and selectable flexure characteristics. Again, the stabilization system 100 is largely similar to the stabilization systems 10 and 80, discussed above. However, the flexure characteristics of the stabilization system 100 of Figs. 8 are principally determined by the cross-sectional size of the spanning structures 102 as a whole between the vertebrae V.

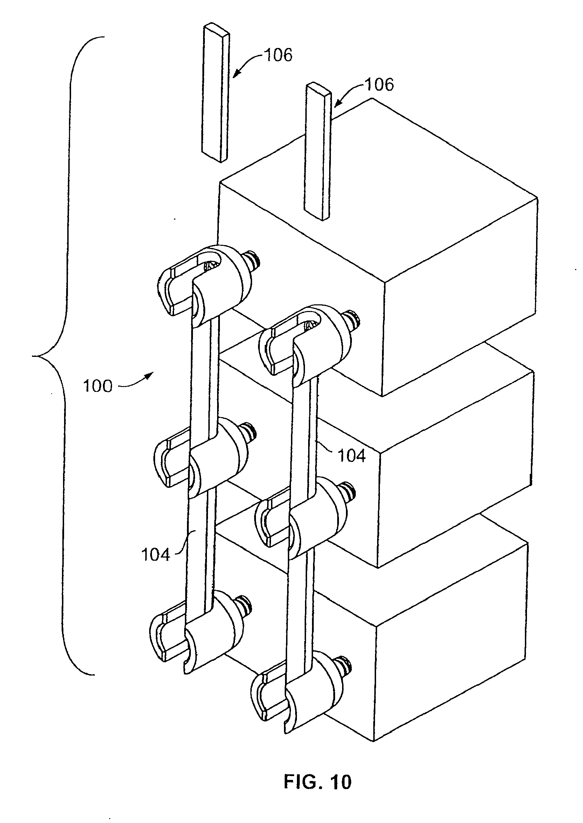

More particularly, a spanning structure 102a between the superior and medial vertebrae VS and VM is approximately twice the cross-sectional size of the spanning structure 102b between the medial and inferior vertebrae VM and VI. As best seen in Fig. 10, the spanning structure 102a, 102b both include portions of a base spanning structure 104 that extends across and between each of the vertebrae V. However, the superior-medial spanning structure 102a additionally includes a secondary spanning structure 106, the combination of the same with the base spanning structure 104 defining the flexure characteristics therefor. Accordingly, the stiffness of the superior-medial spanning structure 102a is greater than the stiffness of the medial-inferior spanning structure 102b.

To the degree each of the spanning structures discussed herein does not exceed its elastic limit (or, more precisely, its change in shape does not exceed, for any portion thereof, a change beyond which deformation becomes permanent), such spanning structures may be modeled as a spring. However, each of the above-discussed forms of the spanning structures provides little, if any, expansion or compression along the longitudinal axial direction of the spanning structures.

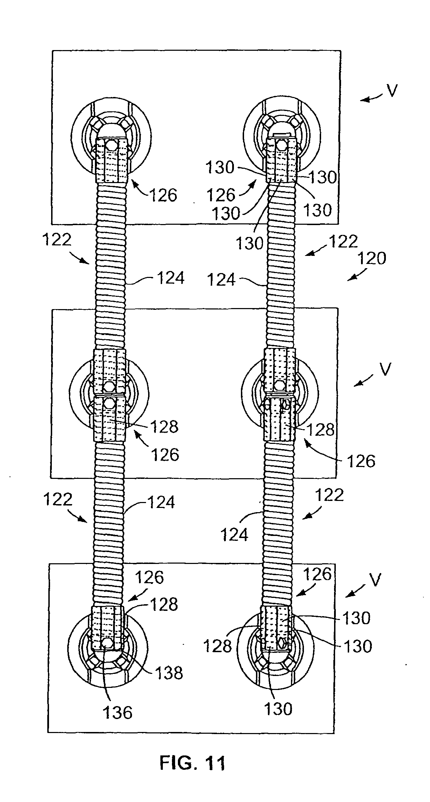

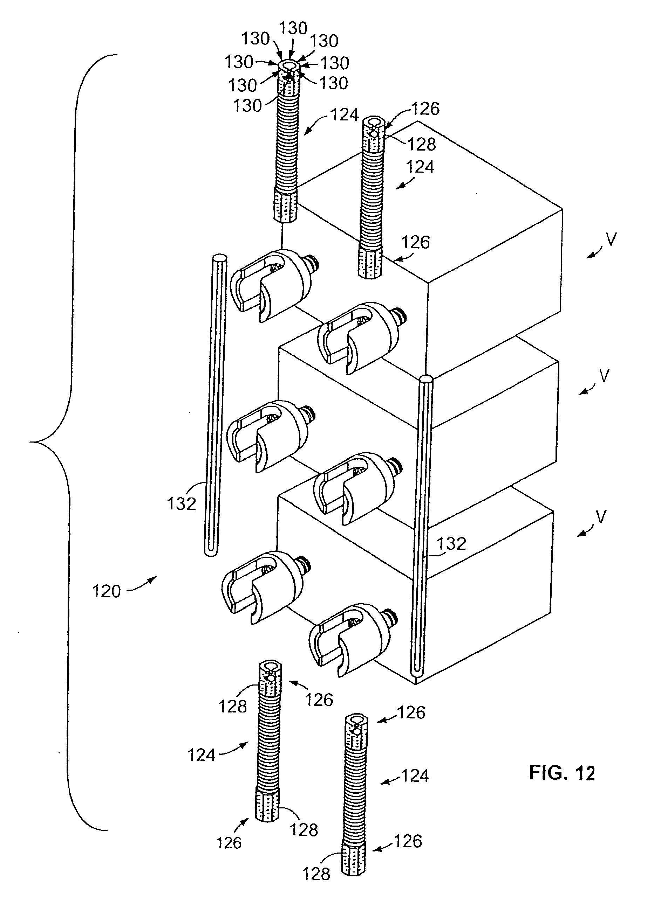

Turning now to Figs. 11 and 12, a further form of a spinal stabilization system 120 is shown having spanning structures 122 that include a coil spring portion 124 that allows the stabilization system 120 to accommodate expansion and contraction of the spanning structure 122 along its longitudinal axis. The stabilization system 120 includes anchors 12 and yokes 18, like each of the above-described embodiments, the spanning structures 122 being received in the yokes 18 and secured therein by a securement such as a cap.

In order to secure the spanning structure 122 with the yokes 18, each end 126 thereof includes an end fixture 128. The end fixture 128 may have any shape, provided that the end fixture 128 is generally sufficiently rigid as to be compressed within the yoke 18 by the securement. The end fixtures 128 are illustrated as being generally octagonal so that flats 130 are formed on the end fixture 128, a pair of the flats 130 contacting the sides of the yoke channel 22, a flat 130 contacting the bottom interior of the yoke channel 22, and a flat 130 being outwardly facing for contact with the cap when secured in the yoke 18. As noted, other configurations of the end fixture 130 may be provided, such as a square or circle; however, the octagonal shape has the benefit of a leading flat 130 that is shorter than the width of the yoke channel 22 to assist in initial advancement of the end fixture 128 into the channel 22. The octagonal shape also provides the benefit of the flats 130 themselves for engaging with the yoke 18 and cap, which serves to provide good compressive contact and serves to retard rotation of the end fixture 128 within the yoke 18 after securement.

Each spanning structure 122 is provided with a single coil spring 124. For the various spanning structures 122 illustrated, each can be provided with varying mechanical performance characteristics. For instance, the effective (i.e., when implanted) spring constant for each coil spring 124 can be selected based on the length of the coil spring 124, a number of turns in the coil spring 124, a diametral size of the coil spring 124, and prestressing of the coil spring 124 when implanted.

A surgeon can easily adjust or alter the performance characteristics by altering the above aspects of the coil spring 124. As best seen in Fig. 11, each end fixture 128 is provided with at least one opening 136. A tool (not shown) can be inserted into the end fixture 128 through an end passage 138 and into the opening 136. The tool can then be used to rotate the end fixture 128 relative to the other end fixture 128, thus pres-stressing the coil spring 124 as well as changing the diametral size and number of coils in the spring 124. In one form, a first of the end fixtures 128 may be positioned in a yoke 18, while the other is manipulated as described. Alternatively or in addition, the first end fixture 128 may be secured in a yoke 18, and the other end fixture 128 may be pulled longitudinally, along the axis of the spanning structure 120, to remove it from the yoke 18; the end fixture 128 may then be rotated and returned within its yoke 18 when the desired number of turns has been made. In order to perform such, loosening of a cap or securement for the end fixture 128 that is rotated may be necessary, particularly if such procedure is performed in a post-operative procedure.

While the spanning structures 122 including the coil springs 124 provide expansion and compression along the longitudinal length, they provide less stiffness in the other directions. Accordingly, a core 132 may be inserted within the coil springs 124. The cores 132 may be provided with varying mechanical performance characteristics, as has been discussed herein, such as by being formed of materials with different elastic moduli.

As shown in Fig. 12, the core 132 may span a plurality of vertebrae V. Alternatively, the cores 132 may span only to two adjacent vertebrae V. In a preferred form, the cores 132 may be removable and replaceable without removal of the securement and end fixtures 128. In this manner, the cores 132 may be changed by the above-described simple incision procedure. Towards this end, the cores 132 may be provided with structure assisting in their removal, such as structure similar to the above-described socket 56 and key 58.

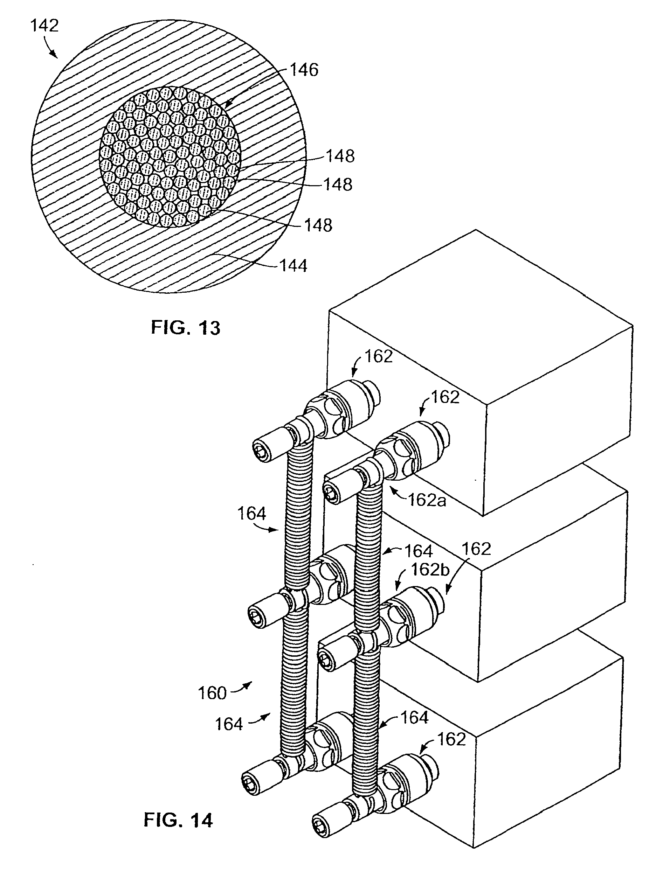

In a form similar to the spanning structures 20 or 122, a stabilization system may be provided with spanning structures 142 that are essentially tubular casings 144, having a hollow bore 146, and a plurality of strands 148 of material are received within the bore 146, as depicted in Fig. 13. The number and/or size of the strands 148 thus cooperate with the casing or sheath 144 to provide the flexure characteristics for the spanning structure 142. In general, the strands 148 would generally be rod or wire-like with a constant diameter and inserted within the casing 144 to provide a desired stiffness. However, the individual strands 148 may also have non-uniform cross-sections, for the reasons discussed herein, and/or may have non-uniform lengths. For the latter, the strands 148 could be staggered or otherwise positioned relative to each other so that the combination of the strands 148 and the casing 144, through any particular cross-section, determine the stiffness thereat.

The number or configuration of the strands 148 may be modified at any desired time, such as post-implantation or post-operatively. That is, it may be convenient to initially implant and secure the casing 144 with the yokes 18, and then insert the strands 148. Furthermore, later minor surgical procedures could be performed to provide additional strands 148, or to remove strands 148, based on the conditions experienced by the patient.

It is known that the bone-screw interface, such as for a pedicle screw, improves over time in the absence (or minimization) of loading on the interface. Therefore, it may be desirable for a portion of the stabilization systems to be implanted with minimal loading on the anchors 12, and a portion to be subsequently adjusted or added to increase the loading on the anchors 12 or the stiffness of the stabilization system.

For instance, the casing 144 may be implanted (or the above-described shell 40 or coil spring 124, for instance) with the bore 146 substantially empty. After a period of time, a minor surgical procedure including a small incision proximate the spanning structure, as is described for Fig. 6, may be performed to increase the stiffness such as by inserting strands 148 into the bore 146.

In a reverse manner, decreasing the stiffness of the spanning structures may be performed in accordance with that discussed for Fig. 6 by making the small incision and removing strands 148 from the bore 146.

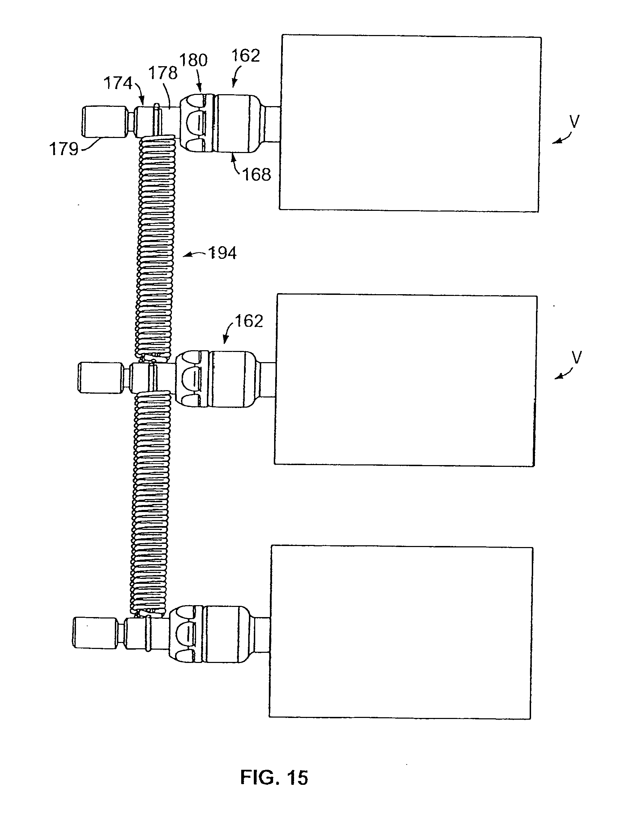

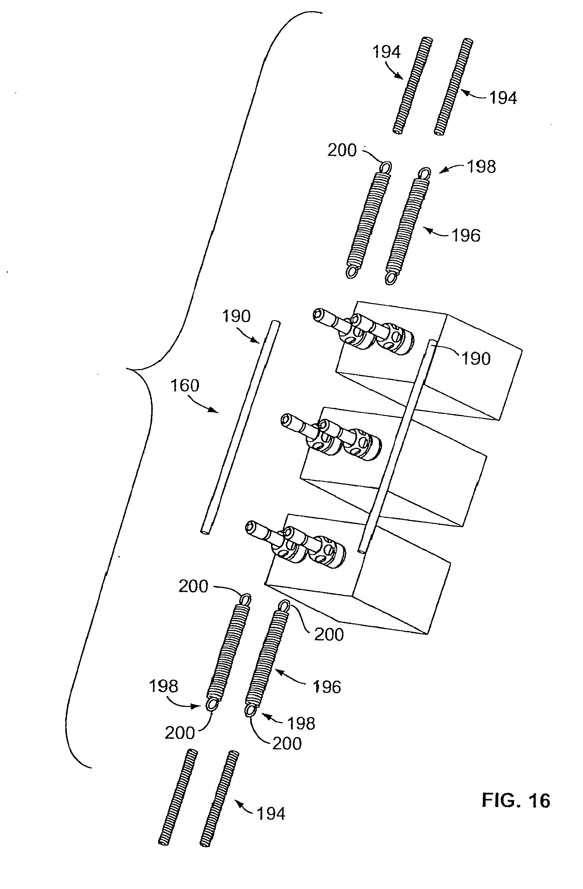

In another form of spinal stabilization system 160, shown in Figs. 14-17, anchors 162 are provided for securing spanning structures 164 having springs. The anchors 162 include a threaded shank 166 as described above and a head 168 which may or may not be polyaxially adjustable, as described. In contrast to the above forms, the head 168 does not form a yoke 18 having a channel 22, instead having a cylindrical recess 170 defined by an upstanding collar 172.

An anchor post 174 cooperates with the head 168 for securing the spanning structures 164 with the anchors 162. The post 174 includes a widened base 176 received in the recess 170 and an upstanding post portion 178. The head collar 172 is threaded (either internally or externally) for receiving a nut 180 thereon for securing the anchor post 174 with the head 168.

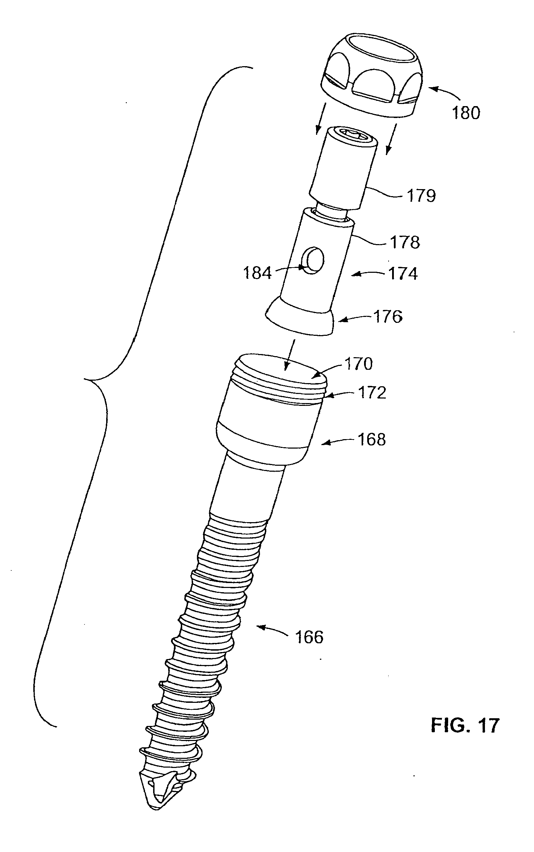

As best seen in Fig. 17, the post portion 178 includes a hollow or a bore 184 into which a portion 190 of the spanning structure 164 is received. Specifically, the portion 190 is a rod-like member linearly advanced through a bore 184 of a first anchor 162a and into a bore 184 of a second anchor 162b, representatively noted in Fig. 14. The post portion 178 receives a set screw 179 that may be driven into the post portion 178 to reach the bore 184 and apply pressure against the portion spanning structure rod 190.

The spanning structures 164 each include a first spring 194 and a second spring 196 located, sheath-like, around the rod portion 190. The first spring 194 has a smaller diameter than the second spring 196 so that the second spring 196 is also positioned, sheath-like, around the first spring 194. The first spring 194 is configured to be compressed from a natural position when the stabilization system 160 is loaded so that the anchors 162 between which the first spring 194 spans are moved toward each other. The second spring 196 is configured to be stretched or expanded from a natural position when the stabilization system 160 is loaded so that the anchors 162 are moved away from each other. In order to maintain the second spring 196 with the anchors 162, an end 198 of each second spring 196 includes an end loop 200 that may be secured around the post portion 178 and, in particular, in an annular groove (not shown) formed in the post portion 178.

As described above, one manner of selectively varying the stiffness of the second (expansion) spring 196 coil is by rotation of the ends 198 to enlarger or contract the diameter of the spring 196, thereby changing its spring equation. It should be noted that the size of the coils may be varied over the length of the spring 196 to give the spring non-linear spring/flexure characteristics. Similarly, the spring properties of the first (compression) spring 194 may be altered.

It should also be noted that the stabilization system 160 may also be adjusted through a small incision formed proximate an anchor 162 in a manner similar to that described for other forms herein. Removal of the rod portion 190 and release of one of the ends 198 of the second spring 196 allows the first spring 194 to be removed and changed, for instance, and the ends 198 may also be subsequently rotated and replaced on the post portion 178.

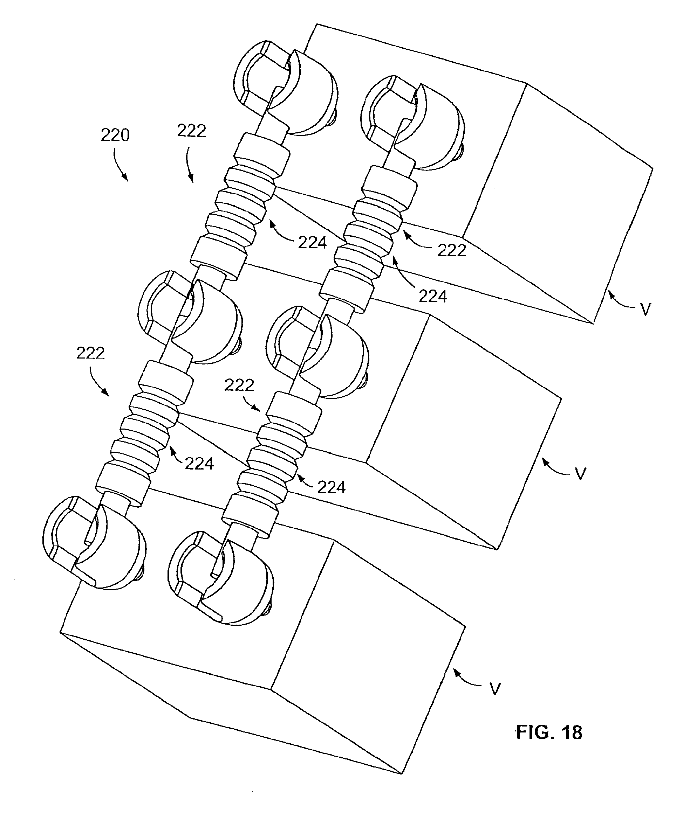

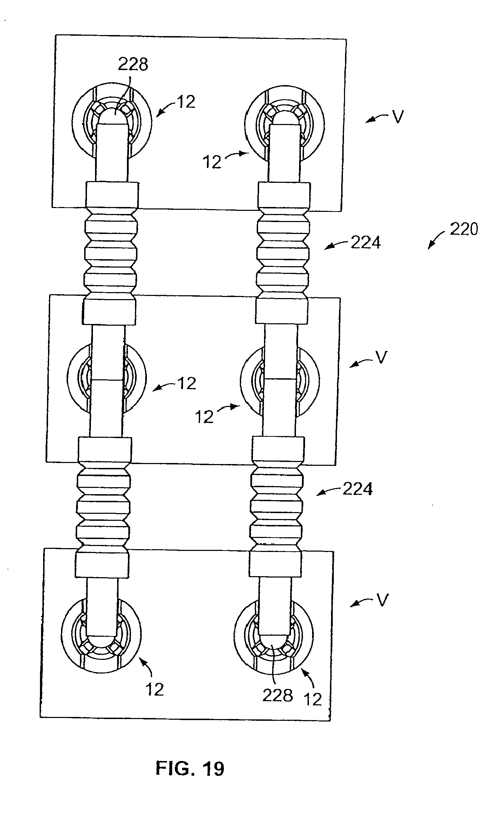

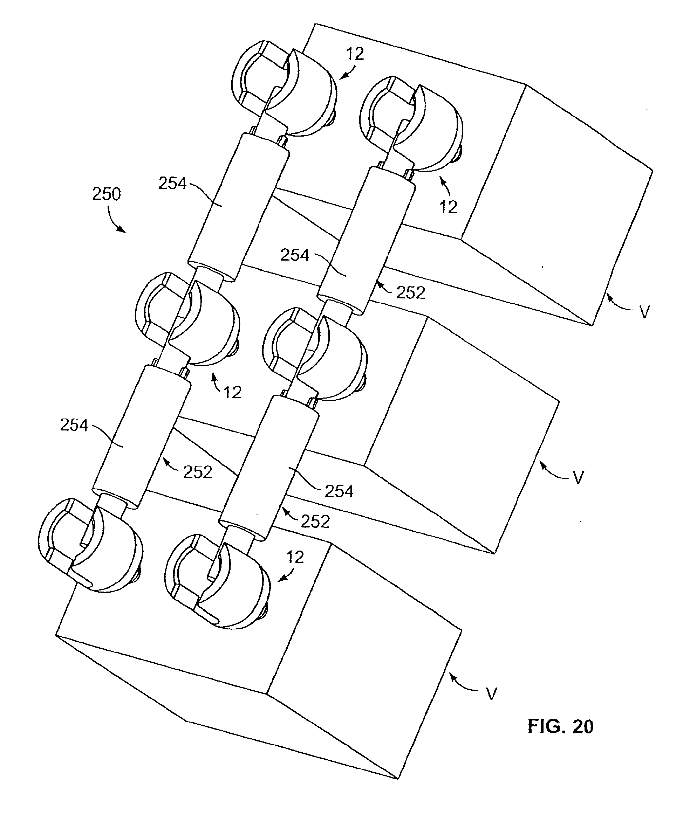

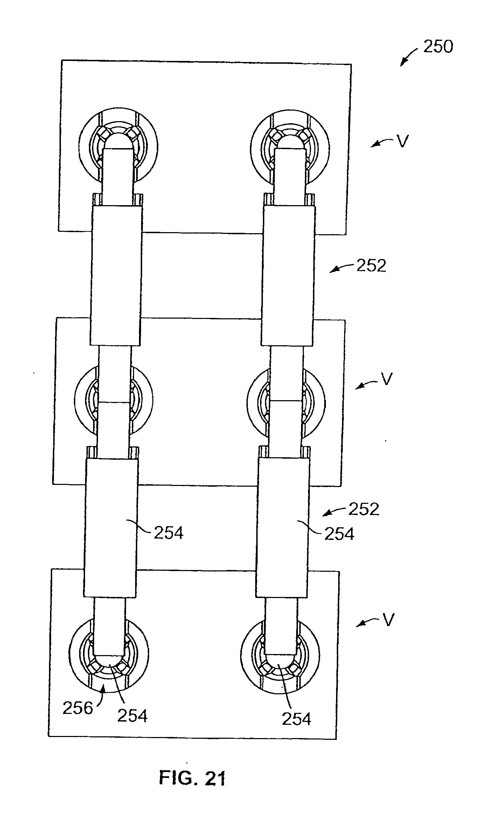

Turning now to Figs. 18-21, forms of spinal stabilization systems are shown using fluid and piston assemblies, fluid referring to both gasses and liquids. As will be discussed in greater detail below, a first form of such systems is shown in Figs. 18 and 19 as stabilization system 220 having a plurality of anchors 12 and spanning structures 222, each having a gas-filled piston 224 assembly thereon. As will also be discussed below, Fig. 20 and 21 depict a stabilization system 250 having a plurality of anchors 12 and spanning structures 252, each having a liquid filled piston assembly 254 thereon.

Turning to Figs. 18 and 19, the piston assembly 224 may be referred to as a pneumatic assembly including a fluid chamber (not shown) and a piston head (not shown) reciprocable within the chamber. The fluid chamber is filled with gas so that movement of the piston head therewithin serves to either compress or expand the gas within the chamber. Accordingly, to some degree, the gas acts as a spring.

The "stiffness" of the gas acting like a spring can be modified by a surgeon user. In a preferred form, an end 226 of each piston assembly 224 includes a port 228 for connection with an external fluid reservoir (not shown) that allows a surgeon to pump in additional fluid or gas, or allows the surgeon to bleed off a portion of the gas. As other embodiments discussed herein, such pressure adjustment may be performed post-operatively, such as through a small incision or via a hypodermic needle injection. Additionally, a reservoir may be implanted subcutaneously that allows for manual pumping of the reservoir, through the skin, and pressure relief. For instance, the reservoir may be a compressible bladder-type device connected via a one-way valve to inject fluid into the piston chamber, and a second one-way valve may be provided for reducing or bleeding fluid from the piston chamber into the bladder.

The stabilization system 220 may be implanted with little or no gas so that the bone-anchor interface is able to heal prior to loading of the stabilization system 220, as has also been discussed above, and subsequently the piston assembly 224 may be pressurized as desired. As can be seen, different piston assemblies 224 of the stabilization system 220 may be provided with different internal pressures within the piston chamber so that each piston assembly 224 has a selected "stiffness."

The stabilization system 250 of Figs. 20 and 21 is similar in operation to that of Figs. 18 and 19. The stabilization system 250 is a hydraulic system utilizing fluid in the form of a liquid that is incompressible or minimally compressible within piston assemblies 252. Accordingly, the the piston assembly 252 is highly resistant to compression or expansion. While this may be viewed as a detriment, it is noted that pumping in or bleeding off of liquid from a port 254 located on an end 256 of the piston assembly 252 provides a high degree of predictability for the performance of the piston assembly 252. In increasing or decreasing the liquid volume, the distance between the anchors 12 to which the piston assembly 252 is secured is relatively easily determined by the surgeon; for instance, a surgeon may be using the stabilization system 250 to relieve pressure on a damage intervertebral disc that is causing pressure and pain on the spinal column, and shifting of vertebrae away from each other by increasing the liquid volume in the piston assembly 252 is evident.

In a variation of the stabilization system 250, the piston assembly 252 may be provided with a dashpot damping structure (not shown) within the fluid (or, more appropriately within the liquid-filled fluid chamber of the piston assembly 252). In this manner, controlled and moderate compression or expansion of the piston assembly 252 is permitted, yet fast or sudden moves are resisted (in proportion to the square of the velocity, as is known in the art). In a further variation, the piston assembly 252 may be provided with an elastically compressible member or material (not shown), either externally located between the piston assembly 252 and an anchor 12 or internally within the piston fluid chamber. In still another variation, the piston assembly 252 may have a fluid of mixed phases, either of same or different material, so that the piston assembly 252 includes the compressibility of a gas form and the incompressibility of a liquid form, and the liquid and gas may be adjusted as desired.

As described, the piston assemblies 224 and 252 may be compressed only in their longitudinal directions, though they would have limited flexibility in other directions. Accordingly, the piston assemblies 224, 252 generally only permit flexure/compression in the anterior-posterior directions. The piston assemblies 224,252 may be calibrated so as to select a desired amount of "stiffness" in their compression. If a compressible fluid were utilized, the "stiffness" may be variable (as opposed to linear based on Boyle's law). Additionally, the fluid may be a non-Newtonian fluid so that shear rate versus force is non-linear, or may have a damper effect by using a fluid of high viscosity and/or internal damper structure. The stiffness characteristics of different piston assemblies in the spinal stabilization systems may vary from assembly to assembly so that, for instance, the stiffness between two vertebral levels may have a first set of characteristics, while the stiffness between two other vertebral levels may have a second set of characteristics.

The above-noted reservoir may, alternatively, be located sub-cutaneously so that post-operative adjustment can be made without revision surgery. In some forms, separate valves may be provided on the piston assemblies for increasing pressure and for decreasing pressure. Additionally, the above-described keys or tools for adjusting the spanning structures or the mechanical performance characteristics thereof may also be joined with the spanning structures and implanted such that non-surgical adjustment of the keys or tools may be had via manipulation through the skin.

It should be noted that, as described, forms of the stabilization system described herein can be adjusted by a simple, relatively straightforward revision procedure, as described for the form of Fig. 6. The spanning portions described herein allow a continuous adjustment and selection (as opposed to an incremented selection based on rod diameter) of the stiffness or modulus of elasticity (or set of characteristics relating thereto). Additionally, spanning portions extending between an inferior vertebra and a second (medial) adjacent vertebra may have a first stiffness, while spanning portions extending between the medial vertebra and an adjacent superior vertebra may have a second stiffness or characteristics relating thereto.

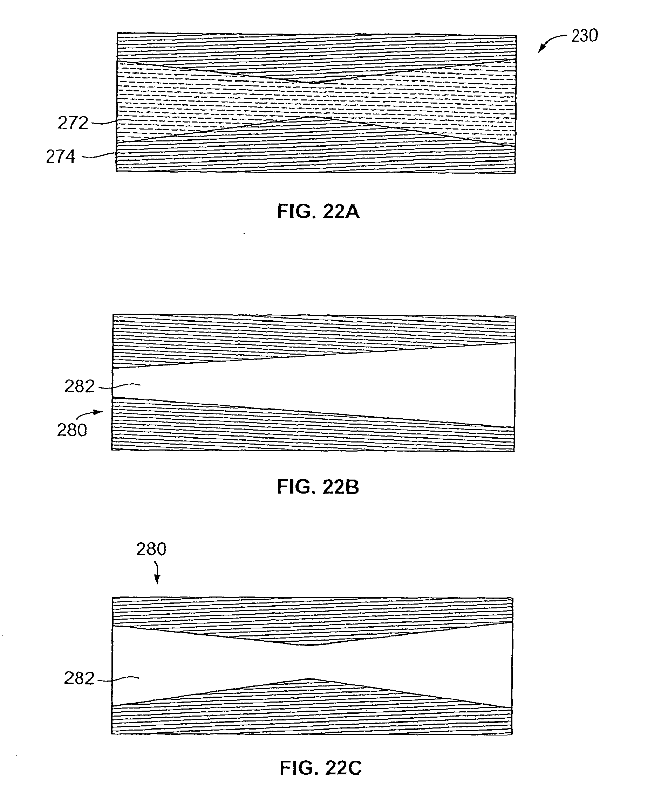

A variety of forms of spanning structures are illustrated in Figs. 22A-22C. A spanning structure 270 may be constructed of various layers of material, two or more of which have differing linear moduli of elasticity. The thickness of the layers may be selected to impart a varying spring equation to the spanning structure 270 over its longitudinal length.

For instance, a central core portion 272 may be formed of material with a first modulus of elasticity, and the central core portion may have a varying cross-sectional shape so that the spring equation for the core portion 272 varies over its longitudinal length. In order to maintain a constant outer diameter to the spanning structure 270, a layer 274 of constant outer diameter may be applied over the core portion, the layer 274 having a varying inner diameter corresponding to the outer diameter of the core portion 272. In this embodiment, the material of the layer portion 274 has an elastic modulus different from that of the core portion 272, and the materials and geometries of the core and layer (or layers) are selected to control or provide a specific set of flexure/bending characteristics.

In another form, a spanning structure 280 may have a hollow core or bore 282 of varying inner diameter. For instance, the bore 282 may have a conical shape (Fig. 21B), a double-frustum shape (Fig. 21C), or another shape. The varying inner diameter allows for the bending of the spanning structure 280 rod to be non-linear proportion to the force applied. In some forms, the above-described scalloping 32, 44 may be formed on the interior surface of the inner bore 282.

It should be noted that any of the above forms may be provided with shock absorbers or the like, such as at an interface between the spanning structures and the anchors. For instance, the spanning structures and the anchors may be joined by an elastomeric or polymeric coupling.

In variations of the present invention, the effective bending characteristics of spanning structures may be varied by varying their geometry, structure, and/or composition. For instance, a single (first) spanning portion may have a varying cross-section over its length, and/or the first spanning portion may have varying cross-section in comparison to a second spanning portion. In some forms, the spanning portions may be constructed as composite or layered member to impart desired flexure characteristics, including varying the thickness or size of layers so that the flexure characteristics are non-linear.

While the invention has been described with respect to specific examples including presently preferred modes of carrying out the invention, those skilled in the art will appreciate that there are numerous variations and permutations of the above described systems and techniques that fall within the spirit and scope of the invention.

Preferred embodiments:
1. A spinal stabilization system securable with a plurality of vertebrae, the system comprising:
   at least one anchor for at each of least two vertebrae;
   a spanning structure extending between and securable with the anchors, wherein the spanning structure has an adjustable mechanical performance characteristic.
2. The system of embodiment 1 wherein the mechanical performance characteristic is a bending stiffness.
3. The system of embodiment 2 wherein the bending stiffness is adjustable in orientation relative to the vertebrae.
4. The system of embodiment 2 wherein the bending stiffness is adjustable in anterior, posterior, lateral, and torsional modes.
5. The system of embodiment 2 wherein the bending stiffness is selected by selection of cross-sectional areas of the spanning structure.
6. The system of embodiment 2 wherein the bending stiffness is selected by selection of differing materials for the spanning structure.
7. The system of embodiment 2 wherein the spanning structure includes an outer member and an inner portion, wherein the bending stiffness is selectable by selection of the inner portion.
8. The system of embodiment 7 wherein the inner portion is provided after securing the outer member with the anchors.
9. The system of embodiment 7 wherein the inner portion is comprised of a plurality of inner components, and the bending stiffness may be selected by selecting a number of the components to be disposed within the outer member.
10. The system of embodiment 9 wherein the bending stiffness is adjustable by removal or addition of the inner components.
11. The system of embodiment 7 wherein the bending stiffness is adjustable by orientation of the inner portion relative to the outer member.
12. The system of embodiment 11 wherein at least one of the outer member and the inner portion having eccentrically positioned regions of reduced cross-sectional area that rotation of the regions provides a direction for lowered stiffness.
13. The system of embodiment 1 wherein the mechanical performance characteristic is a compression/expansion stiffness.
14. The system of embodiment 13 wherein the spanning structure includes a spring including a plurality of coils.
15. The system of embodiment 14 wherein the stiffness is adjustable by adjusting at least one physical characteristic of the spring.
16. The system of embodiment 15 wherein the physical characteristic includes at least one of the number of coils, the diameter of the coils, and the length of the spring.
17. The system of embodiment 14 wherein the coil spring is an outer member, and the spanning structure further includes an inner portion, and wherein the coil spring provides a selectable and adjustable compression/expansion stiffness, and the inner portion provides a bending stiffness.
18. The system of embodiment 14 wherein the spanning structure includes a pair of springs each having a plurality of coils, wherein a first of the springs provides a compression characteristic and a second of the springs provides an expansion characteristic.
19. The system of embodiment 18 wherein the spanning structure further includes an inner portion, wherein one of the springs of the pair forms an outer spring, the other of the springs forms an inner spring, and the inner portion is disposed within the inner spring, the inner portion providing a bending stiffness.
20. The system of embodiment 13 wherein the spanning structure includes a piston assembly compressible and expandable along a longitudinal axis thereof.
21. The system of embodiment 20 wherein the piston assembly is provided with compressible gas.
22. The system of embodiment 20 wherein the piston assembly is provided with substantially incompressible fluid.
23. The system of embodiment 22 wherein the piston assembly is provided with a damper.
24. The system of embodiment 20 wherein the piston assembly is provided with fluid of mixed phases, a portion of the fluid being compressible gas and a portion of the fluid being incompressible liquid.
25. The system of embodiment 20 wherein the piston assembly is provided with fluid, and the amount of fluid is adjustable to adjust the mechanical performance characteristics.
26. The system of embodiment 25 further including a reservoir for the fluid, wherein the piston assembly communicates with the reservoir, and the mechanical performance characteristics adjustable by increasing the fluid in the piston assembly by delivering fluid thereto from the reservoir and is adjustable by decreasing the fluid in the piston assembly by delivering fluid therefrom to the reservoir.
27. The system of embodiment 26 wherein the piston assembly and reservoir are connected via at least two one-way valves for fluid transfer therebetween.
28. The system of embodiment 26 wherein the reservoir is a compressible bladder implanted subcutaneously.
29. A spinal stabilization system securable with a plurality of vertebrae, the system comprising:
   at least one anchor for at each of least two vertebrae;
   a plurality of spanning structures extending between and securable with the anchors, each spanning structure having an adjustable mechanical performance characteristic.
30. The system of embodiment 29 wherein each of the spanning structures is adjusted to impart a different stiffness characteristic between its respective anchors.
31. The system of embodiment 29 wherein the mechanical performance characteristic of the spanning structures adjustable after being secured with the anchors.
32. The system of embodiment 29 wherein the mechanical performance characteristic for at least one of the spanning structures is a bending stiffness, and the mechanical performance characteristic for at least one of the spanning structures is a compression/expansion stiffness.
33. A spinal stabilization system securable with a plurality of vertebrae, the system comprising:
   at least one anchor for at each of least two vertebrae; and
   spanning structures extending between and securable with the anchors, the spanning structure having an adjustable mechanical performance characteristic, wherein the mechanical performance characteristic is adjustable after the spanning structure is secured with its respective anchors.
34. The system of embodiment 33 wherein at least one spanning structure mechanical performance characteristic is adjustable via a percutaneous incision in a patient's skin.
35. The system of embodiment 33 wherein at least one spanning structure is adjustable via an end thereof.
36. The system of embodiment 33 wherein the system is adjustable via an implanted key or tool without an incision.
37. The system of embodiment 33 wherein at least one spanning structure mechanical performance characteristic is adjustable via a hypodermic needle.
38. The system of embodiment 33 wherein at least one spanning structure includes a piston assembly, and the system further including a reservoir for fluid, wherein the piston assembly communicates with the reservoir, the mechanical performance characteristics of the piston assembly being adjustable by increasing the fluid in the piston assembly by delivering fluid thereto from the reservoir and adjustable by decreasing the fluid in the piston assembly by delivering fluid therefrom to the reservoir.
39. The system of embodiment 38 wherein the reservoir is a compressible bladder implanted subcutaneously.

## Claims

1. A spinal stabilization system securable with a plurality of vertebrae, the system comprising:
at least one anchor for each of at least two vertebrae;
a spanning structure extending between and securable with the anchors, wherein the spanning structure has an adjustable mechanical performance characteristic, and
wherein the spanning structure includes a piston assembly compressible and expandable along a longitudinal axis thereof.

2. The system of claim 1 wherein the piston assembly is provided with compressible gas.

3. The system of claim 1 wherein the piston assembly is provided with substantially incompressible fluid.

4. The system of claim 3 wherein the piston assembly is provided with a damper.

5. The system of claim 1 wherein the piston assembly is provided with fluid of mixed phases, a portion of the fluid being compressible gas and a portion of the fluid being incompressible liquid.

6. The system of claim 1 wherein the piston assembly is provided with fluid, and the amount of fluid is adjustable to adjust the mechanical performance characteristics.

7. The system of claim 6 further including a reservoir for the fluid, wherein the piston assembly communicates with the reservoir, and the mechanical performance characteristics adjustable by increasing the fluid in the piston assembly by delivering fluid thereto from the reservoir and is adjustable by decreasing the fluid in the piston assembly by delivering fluid therefrom to the reservoir.

8. The system of claim 7 wherein the piston assembly and reservoir are connected via at least two one-way valves for fluid transfer therebetween.

9. The system of claim 7 wherein the reservoir is a compressible bladder implanted subcutaneously.

10. The system of claim 1 comprising a plurality of spanning structures extending between and securable with the anchors, each spanning structure having an adjustable mechanical performance characteristic.

11. The system of claim 10 wherein each of the spanning structures is adjusted to impart a different stiffness characteristic between its respective anchors.

12. The system of claim 10 wherein the mechanical performance characteristic of the spanning structures adjustable after being secured with the anchors.

13. The system of claim 10, wherein the mechanical performance characteristic is adjustable after the spanning structure is secured with its respective anchors.

14. The system of claim 13 wherein at least one spanning structure mechanical performance characteristic is adjustable via a hypodermic needle.

15. The system of claim 13 wherein at least one spanning structure includes a piston assembly, and the system further including a reservoir for fluid, wherein the piston assembly communicates with the reservoir, the mechanical performance characteristics of the piston assembly being adjustable by increasing the fluid in the piston assembly by delivering fluid thereto from the reservoir and adjustable by decreasing the fluid in the piston assembly by delivering fluid therefrom to the reservoir.

16. The system of claim 15 wherein the reservoir is a compressible bladder implanted subcutaneously.
